(19) Europäisches Patentamt / European Patent Office / Office européen des brevets

(11) **EP 1 759 627 B1**

(12) **EUROPEAN PATENT SPECIFICATION**

(45) Date of publication and mention
of the grant of the patent:
**04.12.2013  Bulletin 2013/49**

(51) Int Cl.:
***A61B 1/00*** *(2006.01)*

(21) Application number: **06018149.2**

(22) Date of filing: **30.08.2006**

(54) **Hood for endoscope, endoscope and method of fixing balloon for endoscope**

Haube für ein Endoskop, Endoskop und Verfahren zur Ballonfixation auf einem Endoskop

Endoscope, capuchon pour endoscope et méthode de fixation d un ballon pour endoscope

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **31.08.2005  JP 2005251931**

(43) Date of publication of application:
**07.03.2007  Bulletin 2007/10**

(73) Proprietor: **FUJIFILM Corporation
Tokyo (JP)**

(72) Inventor: **Fujikura, Tetsuya
Saitama-shi, Saitama (JP)**

(74) Representative: **Klunker . Schmitt-Nilson . Hirsch
Patentanwälte
Destouchesstrasse 68
80796 München (DE)**

(56) References cited:
**EP-A1- 0 112 148     EP-A2- 0 283 661
DE-A1- 3 131 652     US-A- 4 960 106
US-A- 5 681 260     US-A- 6 059 719
US-B1- 6 306 081**

**Description**

BACKGROUND OF THE INVENTION

1. Technical Field

The invention relates to an endoscope according to the preamble of claim 1. More particularly, the invention relates to an endoscope that includes a hood for the endoscope and a balloon for the endoscope attached to an end of an insertion part.

2. Description of Related Art

In the case where an insertion part of an endoscope is inserted in the deep alimentary canal such as the small intestine, it is difficult to apply a force to an end of the insertion part by merely pushing the insertion part because of complicated bents of the intestinal canal. Therefore, it is difficult to insert the insertion part in the deep part. If the insertion part is excessively bent or curved, it is still more difficult to insert the insertion part into the deep part. Therefore, a method of preventing the insertion part of the endoscope from being bent or curved has been proposed in which the insertion part of the endoscope is inserted while being covered with an insertion subsidiary member, and the insertion subsidiary member guides the insertion part.

For example, JP 2002-301019 A discloses an endoscope device that includes a first balloon attached to an end of an insertion part of the endoscope and a second balloon attached to an end of an insertion subsidiary member (may be referred to as an over tube or a sliding tube). The first balloon and the second balloon may inflate to fix the insertion part or the insertion subsidiary member to the intestinal canal such as the small intestine. Accordingly, the first balloon and the second balloon may inflate and deflate repeatedly to alternately insert the insertion part and the insertion subsidiary member. Therefore, it is possible to insert the insertion part into the deep part of intestinal canal, such as the small intestine, which bends complexly.

Typically, the balloon for the endoscope that is used for the above-mentioned purpose is formed of thin silicone rubber or latex and has a cylinder shape. The balloon is put on the insertion part of the endoscope, and for example, a cylindrical rubber band is then fitted to both ends of the balloon to fix the balloon in the vicinity of the end of the insertion part.

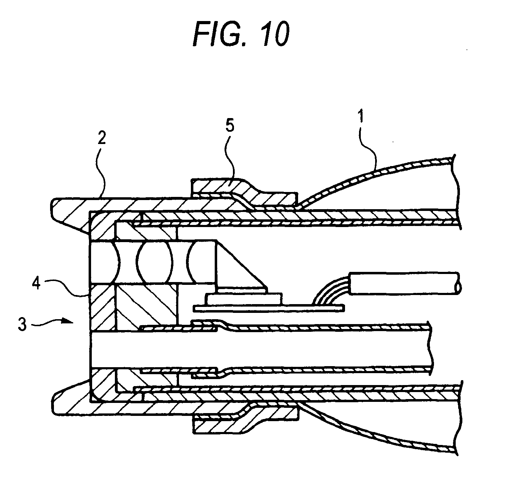

However, as shown in Fig. 10, a hood 2 for an endoscope as well as a balloon 1 for the endoscope may be attached to an insertion part of the endoscope. The hood 2 for the endoscope is used to maintain a distance between an object to be observed and an end 3 of the endoscope, or to suppress movement of the object caused by pulsation of the internal organ. The hood 2 for the endoscope, which is used for the above-mentioned purpose, generally has a cylinder shape, and is fitted to the end 3 of the insertion part so as to protrude a predetermined length from an end face 4 of the end 3.

However, in the case where the hood 2 for the endoscope and the balloon 1 for the endoscope are attached to the end 3 of the insertion part, since a band 5, which fixes the balloon 1 for the endoscope, overlaps the hood 2 for the endoscope, an outer diameter of the overlapped portion may be increased, and the hood 2 for the endoscope or the balloon 1 for the endoscope may not be fixed sufficiently. Furthermore, since the hood 2 for the endoscope and the band 5 interfere with each other, operation of attaching the hood 2 for the endoscope or the balloon 1 for the endoscope may be difficult.

In accordance with the preamble of claim 1, US-A-5,681,260 (figure 11 thereof) discloses an endoscope in which a hood having a spherical form comprises an extension to which an end portion of the balloon is attached by means of a fastening member similar to a band. The hood comprises an inner winding complementary to an outer winding provided at the tip end of the insertion part of the endoscope. This allows to fasten the hood to the insertion part of the endoscope.

SUMMARY OF THE INVENTION

Accordingly, the invention has been made in view of the above circumstances, and provides an endoscope where the hood for the endoscope and a balloon for the endoscope can be attached surely and a diameter of an insertion part of the endoscope can be reduced after the hood and the balloon are attached.

According to an aspect of the invention, an endoscope has the features of claim 1.

According to this structure, the hood of the endoscope includes the balloon fixing part. Therefore, the balloon for the endoscope can be fixed by the hood for the endoscope. Accordingly, since it becomes unnecessary to use a member for fixing the balloon for the endoscope (for example, a band) for the endoscope, the number of parts can be reduced. Furthermore, since the member for fixing the balloon for the endoscope and the' hood for the endoscope do not overlap, the diameter of the insertion part of the endoscope after attachment can be reduced. Furthermore, according to the above structure, the balloon for the endoscope and the hood for the endoscope can be attached simultaneously. Thus, the attachment operation can be performed for a short time.

According to claimed structure, since the inner diameter of the balloon fixing part is smaller, a force tightening the balloon for the endoscope is increased. As a result, the balloon for the endoscope can be surely fixed.

Also, the hood for the endoscope may further include a holder at an end of the hood, wherein the holder can hold a medical capsule. Accord to this structure, the medical capsule can be conveyed while the end of the hood is holding the capsule.

According to this structure, since the balloon for the endoscope is fixed by the hood for the endoscope, it is unnecessary to use a member (for example, a band) for fixing the balloon for the endoscope. Accordingly, the number of parts can be reduced and the diameter of the insertion part of the endoscope after attachment can be reduced.

According to this structure, since the balloon for the endoscope and the hood for the endoscope are formed integrally, the balloon for the endoscope and the hood for the endoscope can be attached simultaneously. Accordingly, the attachment operation can be performed easily. Furthermore, since it is unnecessary to use a member for fixing the hood for the endoscope, the number of parts can be reduced and the diameter of the insertion part of the endoscope after the attachment can be reduced.

According to this configuration, since the balloon for the endoscope is fixed using the hood for the endoscope, it is unnecessary to separately use a member for fixing the balloon for the endoscope. Accordingly, the diameter of the insertion part of the endoscope after attachment can be reduced. Further, since the hood for the endoscope and the balloon for the endoscope are attached simultaneously, the attachment operation can be performed easily.

According to the invention, since a balloon for an endoscope is fixed by a hood for an endoscope, it is possible to reduce the number of parts. Furthermore, an operation of attaching the balloon for the endoscope and the hood for the endoscope can be performed for a short time. Also, a diameter of an insertion part of the endoscope after attachment of the hood and the balloon can be reduced.

BRIEF DESCRIPTION OF THE DRAWINGS

Fig. 1 is a view illustrating a system of an endoscope device according to an embodiment of the invention.
Fig. 2 is a perspective view of an end of an insertion part of the endoscope.
Fig. 3 is an exploded view showing a configuration of the end of the insertion part and a hood.
Fig. 4 is a sectional view of the end of the insertion part to which the hood of Fig. 3 is attached.
Fig. 5 is a view explaining an method for operating the endoscope device according to the embodiment of the invention.
Fig. 6 is a view explaining a hood having another shape.
Fig. 7 is a sectional view of the hood having another shape.
Fig. 8 is a sectional view of the hood having still another shape.
Fig. 9 is a sectional view of a hood according to another embodiment of the invention.
Fig. 10 is a sectional view of an endoscope according to a related art.

DETAILED DESCRIPTION OF EXEMPLARY EMBODIMENTS

Hereinafter, a hood for an endoscope, an endoscope, and a method of fixing a balloon for an endoscope according to exemplary embodiments of the invention will be described in detail with reference to accompanying drawings.

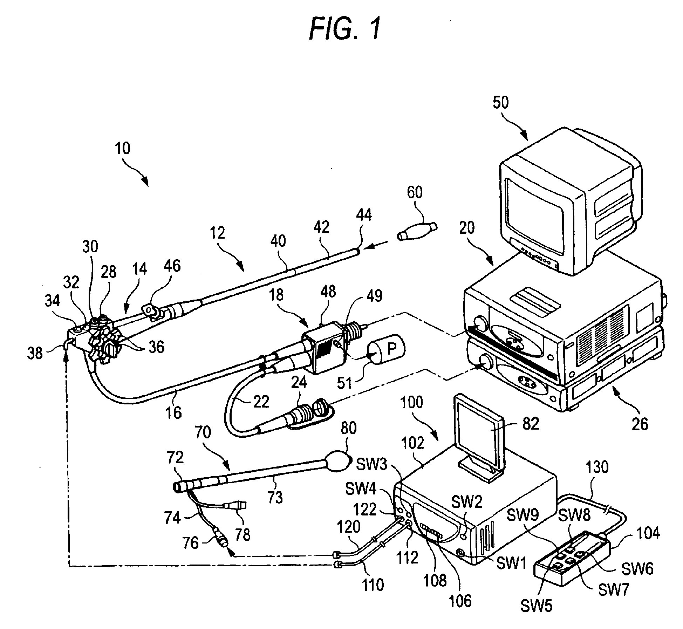

Fig. 1 is a view illustrating a system configuration of an endoscope device of a double balloon type according to an embodiment of the invention. As shown in Fig. 1, the endoscope device includes an endoscope 10, an insertion subsidiary member 70, and a balloon control unit 100.

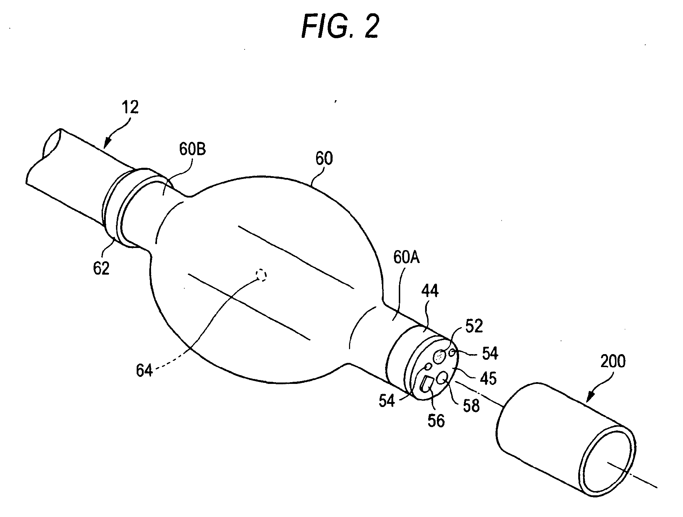

The endoscope 10 includes a hand operation part 14 and an insertion part 12, which is connected to the hand operation part 14 and is to be inserted into the coelom. A universal cable 16 is connected to the hand operation part 14. An LG connector 18 is provided on a forward end of the universal cable 16. The LG connector 18 is detachably connected to a light source device 20. Light is transmitted through the LG connector to an illumination optical system 54 as described later (see Fig. 2). Furthermore, an electric connector 24 is connected to the LG connector 18 through a cable 22. The electric connector 24 is detachably connected to a processor 26.

An air-and-water supply button 28, a suction button 30, a shutter button 32, and a function change button 34 are provided side by side on the hand operation part 14. A pair of angle knobs 36 and 36 is also provided on the hand operation part 14. A pipe, which is bent in an L-shape, is formed as a balloon air supply port 38 on a base end of the hand operation part 14. Fluid such as air is supplied to or suctioned from the balloon air supply port 38 to inflate or deflate a first balloon 60 as described later.

The insertion part 12 includes a soft portion 40, a curved portion 42, and a forward end 44 in order from the hand operation part 14. The angle knobs 36 and 36 of the hand operation part 14 are rotated to remotely curve the curved portion 42. Accordingly, the forward end 44 can be oriented in a desired direction.

As shown in Fig. 2, an observation optical system 52, illumination optical systems 54 and 54, an air-and-water supply nozzle 56, and a clamp opening (corresponding to a suction opening) 58 are provided on a front face 45 of the forward end 44.

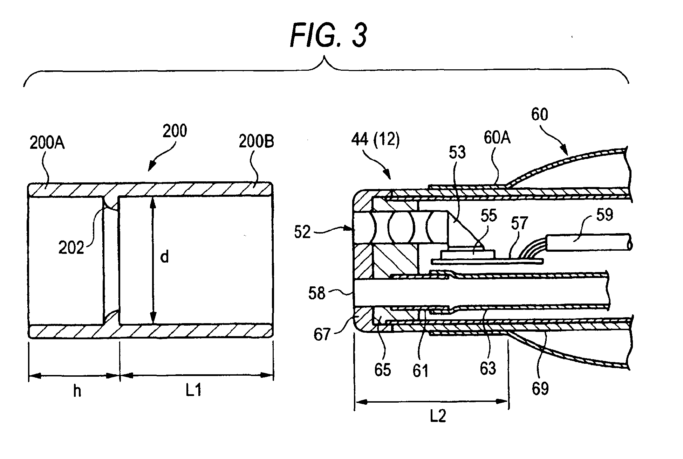

A prism 53 shown in Fig. 3 is provided in the rear of the observation optical system 52. A path of light, which is received from an object through the observation optical system 52 is bent downward by the prism 53. A CCD 55, which is supported by a board 57, is disposed under the prism 53 so that light from the object bent by the prism 53 forms an image on a light receiving surface of the CCD 55. The CCD 55 converts light from the object, into an electric signal. The electric signal is transmitted through a signal cable 59. The signal cable 59 is inserted into the insertion part 12, the hand operation part 14 and the universal cable 16 shown in Fig. 1, extends to the electric connector 24, and is connected to the processor 26. Therefore, an observed image captured through the observation optical system 52 is formed on the light receiving surface of the CCD 55 and then converted into the electric signal. The electric signal is output through the signal cable 59 into the processor 26 and then converted into a video signal. Thereby, the observed image is displayed on a monitor 50 connected to the processor 26.

An emission port of a light guide (not shown) is disposed in the rear of the illumination optical systems 54 and 54 of Fig. 2. The light guide is inserted into the insertion part 12, the hand operation part 14, and the universal cable 16, which are shown in Fig. 1, and an entrance port of the light guide is disposed in the LG connector 18. Accordingly, the LG connector 18 is connected to the light source device 20, and illumination light is transmitted from the light source device 20 to the illumination optical systems 54 and 54 through the light guide, and is emitted forward from the illumination optical systems 54 and 54.

The air-and-water supply nozzle 56 shown in Fig. 2 communicates with a valve (not shown), which is operated by the air-and-water supply button 28 shown in Fig. 1. The valve communicates with an air-and-water supply connector 48 provided in the LG connector 18. An air-and-water supply unit, which is not shown, is connected to the air-and-water supply connector 48 to supply air or water. Accordingly, when the air-and-water supply button 28 is pushed, air or water ejected from the air-and-water supply nozzle 56 to the observation optical system 52.

The clamp opening 58 shown in Fig. 2 communicates with a pipe 61 supported by an end-part main body 65 shown in Fig. 3. Furthermore, a tube 63 is connected to the pipe 61. The tube 63 is inserted into and disposed in the insertion part 12 shown in Fig. 1 to communicate with a clamp insertion part 4 6. Accordingly, when a medical device such as clamp is inserted through the clamp insertion part 46, the medical device can be drawn from the clamp opening 58. Additionally, the tube 63 shown in Fig. 3 is branched, and the branched portion communicates with a valve (not shown), which is operated by the suction button 30 shown in Fig. 1. This valve is further connected to a suction connector 49 of the LG connector 18. A suction pump 51 is connected to the suction connector 49. Therefore, when the suction pump 51 is operated and the valve is operated using the suction button 30, body fluid or air can be suctioned through the clamp opening 58.

Further, in Fig. 3, reference numeral 67 denotes a cap provided on the front face of the end-part main body 65. Also, reference numeral 69 denotes an outer-coat member covering a circumferential surface of the insertion part 12.

As shown in Fig. 2, a first balloon 60 formed of an elastic body, such as rubber, is provided on the circumferential surface of the insertion part 12. The first balloon 60 is formed into a substantial tube shape where both ends 60A and 60B are reduced in diameter. The insertion part 12 is inserted into the first balloon 60 and then, the first balloon 60 is fixed at a predetermined position. A fixing ring 62 formed of rubber is fitted to a rear end 60B of the first balloon 60 to fix the rear end 60B of the first balloon 60. Also, an endoscope hood 200, which will be described later, fixes the forward end 60A of the first balloon 60.

An air hole 64 is formed in a portion of the circumferential surface of the insertion part 12 to which the first balloon 60 is attached. The air hole 64 communicates with a balloon-air supply opening 38 provided on the hand operation part 14 shown in Fig. 1. The balloon-air supply opening 38 communicates with a balloon control unit 100 through a tube 110. Accordingly, when air is supplied or suctioned using the balloon control unit 100, the first balloon 60 can be inflated or deflated. When supplied with air, the first balloon 60 inflates to have a substantially spherical shape. Also, when air is suctioned, the first balloon 60 is brought into contact with the circumferential surface of the insertion part 12.

Meanwhile, the insertion subsidiary member 70 shown in Fig. 1 includes a hard gripper 72 and a main-body tube 73. The gripper 72 is provided on a base-end side of the insertion subsidiary member 70 and has a tube shape. The main-body tube 73 is provided on a forward-end side of the gripper 72. The insertion part 12 of the endoscope 10 is inserted through the gripper 72 into the main-body tube 73.

The main-body tube 73 includes a flexible resin tube, which is made of urethane, as a base member. Inner and outer surfaces of the base member are coated with a hydrophilic coating material (lubricative coating material). Examples of the hydrophilic coating material may include polyvinyl pyrrolidone, an acryl resin, and a silicone resin.

A second balloon 80 is provided in the vicinity of the forward end of the main-body tube 73. The second balloon 80 is formed into a tube shape where both ends are reduced in diameter. The second balloon 80 is attached to the insertion subsidiary member 70 with the insertion subsidiary member 70 passing therethrough. A thread, which is not shown, is wound to fix the second balloon 80 to the insertion subsidiary member 70. The second balloon 80 communicates with a tube 74 attached to the outer surface of the insertion subsidiary member 70. A connector 76 is provided on a base end of the tube 74. A tube 120 is connected to the connector 76. Thus, the connector 76 is connected to the balloon control unit 100 through the tube 120. Accordingly, when air is supplied or suctioned using the balloon control unit 100, the second balloon 80 is inflated or deflated. When supplied with air, the second balloon 80 is inflated to have a substantially spherical shape. Also, when air is suctioned, the second balloon 80 is brought into contact with the circumferential surface of the insertion subsidiary member 70.

An injection opening 78 is provided on the base end of the insertion subsidiary member 70. The injection opening 78 communicates with an opening (not shown) formed in the inner surface of the insertion subsidiary member 70. Therefore, when a lubricating agent (for example, water) is injected through the injection opening 78 using a syringe, the lubricating agent can be supplied into the insertion subsidiary member 70. Accordingly, when the insertion part 12 is inserted into the insertion subsidiary member 70, a friction between the inner surface of the insertion subsidiary member 70 and the outer surface of the insertion part 12 can be reduced. Thus, the insertion part 12 can move smoothly with respect to the insertion subsidiary member 70.

The balloon control unit 100 supplies fluid, such as air, to the first balloon 60 or suctions the fluid from the first balloon 60. Also, the balloon control unit 100 supplies the fluid, such as air, to the second balloon 80 or suctions the fluid from the second balloon 80. The balloon control unit 100 includes a main body 102 and a hand switch 104 for remote control.

A power switch SW1, a stop switch SW2, a first pressure display part 106, a second pressure display part 108, a first function stop switch SW3, and a second function stop switch SW4 are provided on a front face of the main body 102. The first pressure display part 106 and the second pressure display part 108 are panels that display pressures of the first balloon 60 and the second balloon 80, respectively. When an abnormal situation occurs, for example, when the balloon pops, an error code is displayed on the pressure display parts 106 and/or 108.

The first function stop switch SW3 and the second function stop switch SW4 are switches that have ON/OFF functions with respect to a control system A for the endoscope and a control system B for the insertion subsidiary member as described later. If only any one of the first balloon 60 and the second balloon 80 is used, the function stop switch SW3 or SW4 for another balloon, which is not used, is operated to turn off its function. In the control system A or B that is turned off, supply and suction of air are completely stopped, and the corresponding pressure display part 106 or 108 of the system is also turned off. When both of the function stop switches SW3 and SW4 are turned off, settings for the initial state can be set up. For example, when both of the function stop switches SW3 and SW4 are turned off and the switches SW5 to SW9 of a hand switch 104 are simultaneously pushed, calibration with respect to atmospheric pressure is performed.

The tube 110 through which air is supplied into the first balloon 60 or suctioned from the first balloon 60, and the tube 120 through which air is supplied into the second balloon 80 or suctioned from the second balloon 80 are connected to the front face of the main body 102. Backflow prevention units 112 and 122 are provided at connection portions between the tubes 110 and 120 and the main body 102 so as to prevent body fluid from flowing backward when the first balloon 60 or the second balloon 80 are popped. Each of the backflow prevention units 112 and 122 are configured that a gas-liquid separation filter is incorporated into a casing having a hollow disk shape (not shown), which is detachably provided on the main body 102. Each of the backflow prevention units 112 and 122 prevents liquid from flowing into the main body 102 using the filter.

The pressure display parts 106 and 108, the function stop switches SW3 and SW4, and the backflow prevention units 112 and 122 for the endoscope 10 and the insertion subsidiary member 70 are provided at predetermined locations. That is, the pressure display part 106, the function stop switch SW3 and the countercurrent breaking unit 112 for the endoscope 10 are disposed on the right side of the pressure display part 108, the function stop switch SW4 and the backflow prevention unit 122 for the insertion subsidiary member 70, respectively.

Meanwhile, the hand switch 104 is provided with the stop switch SW5 like the stop switch SW2 of the main body 102; the ON/OFF switch SW6, which orders application of pressure to the first balloon 60 and reduction of pressure in the first balloon 60; the pause switch SW7 for maintaining pressure of the first balloon 60; the ON/OFF switch SW8, which orders application of pressure to the second balloon 80 and reduction of pressure in the second balloon 80; and the pause switch SW9 for maintaining pressure of the second balloon 80. The hand switch 104 is electrically connected to the main body 102 through a cord 130. Furthermore, a display part, which is not shown in Fig. 1, is provided on the hand switch 104 to display supplying state/discharging state of air to/from the first balloon 60 or the second balloon 80.

The balloon control unit 100 having the abovementioned configuration supplies air to each of the balloons 60 and 80 to inflate each of the balloons 60 and 80and while controlling air pressure at a predetermined value to maintain the inflation of each of the balloons 60 and 80. Additionally, the balloon control unit 100 suctions air from each of the balloons 60 and 80 to deflate each of the balloons 60 and 80 while controlling air pressure at a predetermined value to maintain the deflation of each of the balloons 60 and 80.

The balloon control unit 100 is connected to a monitor 82 dedicated to the balloon. When each of the balloons 60 and 80 inflates or deflates, pressure values or the inflation state/deflation state of each of the balloons 60 and 80 is displayed on the monitor 82 dedicated to the balloon. The pressure values or the inflation state/deflation state of each of the balloons 60 and 80 may be superimposed on an observation image of the endoscope 10 and then displayed on the monitor 50.

As shown in Fig. 2, a hood 200 is fitted to the forward end 44 of the insertion part 12 of the endoscope 10. The hood 200 is formed of an elastic material, such as silicone rubber, to have a cylinder shape. Also, when the hood 200, which is transparent or semitransparent, is attached to the forward end 44 of the insertion part 12, an outside of the hood 200 can be observed using an observation optical system 52.

As shown in Fig. 3, an inner diameter d of the hood 200 is slightly smaller than an outer diameter of the forward end 44 of the insertion part 12. The hood 200 is attached to the forward end 44 while the base end 200B of the hood 200 is being elastically deformed.

A position regulation portion 202 protrudes from an inner surface of the hood 200, and comes into contact with a front face 45 of the insertion part 12 when the hood 200 is attached to the forward end 44. Accordingly, when the hood 200 is attached to the forward end 44, a protrusion height h of the hood 200 is set to be a predetermined value. It is preferable that the protrusion height h and inner diameter d of the hood 200 satisfy the following equation with respect to a radius r of a hemisphere portion of a medical capsule 220.

$$h + \sqrt{r^2 - \frac{d^2}{4}} > r \qquad (1)$$

The forward end 200A of the hood 200 is formed so as to satisfy the above-mentioned equation. Thereby, when the forward end 200A of the hood 200 suctions the medical capsule 220, the medical capsule 220 does not come into contact with the front face 45 of the insertion part 12. Accordingly, since the medical capsule 220 comes into close contact with the forward end 200A of the hood 200, pressure in the hood 200 is reduced when air is suctioned through the clamp opening 58. Thereby, the medical capsule 220 can be suctioned and held surely. That is, when the forward end 200A of the hood 200 satisfies the above-mentioned equation, the forward end 200A of the hood 200 can be formed with a holder for the medical capsule 220. Further, the inner diameter d of the opening and protrusion height h of the hood 200 may vary according to the shape of the medical capsule 220 (that is, the dimension of r).

Additionally, in the hood 200, a length L1 between the position regulation portion 202 and its base end is equal to a length L2 between the front face 45 of the insertion part 12 and the forward end 60A of the first balloon 60. Therefore, when the base end 200B of the hood 200 is fitted to the forward end 44 of the insertion part 12, the base end 200B of the hood 200 is fitted to the forward end 60A of the first balloon 60. Thus, the hood 200 tightens and fixes the forward end 60A of the first balloon 60. That is, the hood 200 is provided so that the base end 200B extends to a position where the forward end 60A of the first balloon 60 is attached, so that the base end 200B is formed with a part for fixing the first balloon 60.

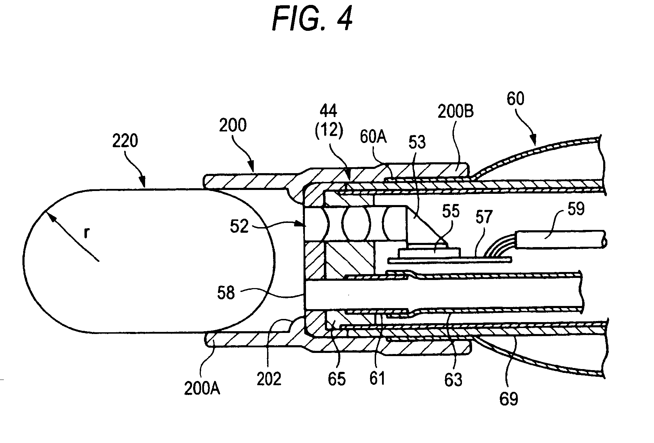

The hood 200 and the first balloon 60 having the above-mentioned configuration are attached to the insertion part 12 of the endoscope 10 in the following manner. First, as shown in Fig. 2, the insertion part 12 is inserted into the first balloon 60, and the first balloon 60 is disposed at a predetermined position in the vicinity of the forward end 44 of the insertion part 12. A fixing ring 62 is fitted to the rear part 60B of the first balloon 60, and the rear part 60B of the first balloon 60 is fixed into the insertion part 12. Subsequently, as shown in Fig. 4, the hood 200 is fitted to the front face 45 of the insertion part 12, and the base end 200B of the hood 200 is fitted to the forward end 60A of the first balloon 60. Accordingly, the base end 200B of the hood 200 tightens and fixes the forward end 60A of the first balloon 60.

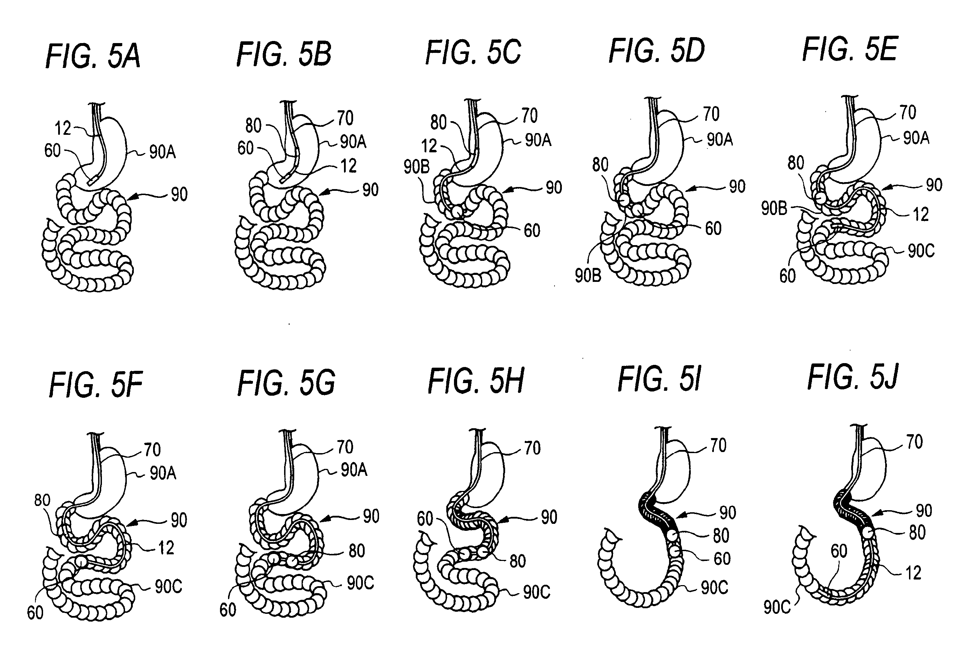

Next, a method of inserting the insertion part 12 of the endoscope device having the above-mentioned configuration into the coelom will be described with reference to Figs. 5A to 5J. It is noted that Figs. 5A to 5J illustrate oral insertion, but anal insertion may be conducted.

First, while the first balloon 60 and the second balloon 80 deflate, the insertion part 12 is inserted into the insertion subsidiary member 70 and then, it is started to insert the insertion part 12. As shown in Fig. 5A, the insertion part 12 is inserted until the forward end of the insertion part 12 reaches the stomach 90A. Subsequently, the insertion subsidiary member 70 is inserted along the insertion part 12. As shown in Fig. 5B, the forward end of the insertion subsidiary member 70 reaches the stomach 90A.

Next, while the insertion subsidiary member 70 is gripped so as not to be withdrawn from the coelom, the insertion part 12 is pushed into the inside of the insertion subsidiary member 70 and is inserted until the forward end of the insertion part 12 reaches the limb 90B of the duodenum as shown in Fig. 5C (insertion operation). Additionally, the first balloon 60 is inflated to fix the forward end of the insertion part 12 to the limb 90B of the duodenum (fixing operation).

Subsequently, the insertion subsidiary member 70 is pushed so as to insert the insertion subsidiary member 70 along the insertion part 12 (pushing operation). Further, as shown in Fig. 5D, after the forward end of the insertion subsidiary member 70 is brought in the vicinity of the first balloon 60, air is supplied into the second balloon 80 to expand the second balloon 80. Accordingly, the second balloon 80 is fixed to the descending limb 90B of the duodenum, and the insertion subsidiary member 70 holds the limb 90B of the duodenum through the second balloon 80 (holding operation).

In this state, the insertion subsidiary member 70 and the insertion part 12 are manually drawn at the same time (manual drawing operation). Accordingly, excessive flexion or bending of the alimentary canal 90 to the descending limb 90B of the duodenum can be eliminated.

Subsequently, air is suctioned from the first balloon 60 to deflate the first balloon 60. As shown in Fig. 5E, the insertion part 12 is then inserted into the inside of the small intestine 90C (insertion operation). At this time, since the excessive flexion of the alimentary canal 90 to the descending limb 90B of the duodenum has been eliminated by the insertion subsidiary member 70, the insertion part 12 is inserted easily.

Next, as shown in Fig. 5F, the first balloon 60 expands to fix the forward end of the insertion part 12 to the alimentary canal 90. After the second balloon 80 deflates, as shown in Fig. 5G, the insertion subsidiary member 70 is inserted along the insertion part 12, and the second balloon 80 is inflated in a state where the forward end of the insertion subsidiary member 70 is close to the first balloon 60.

Next, as shown in Fig. 5H, while the first balloon 60 and the second balloon 80 are inflated, the insertion subsidiary member 70 and the insertion part 12 are manually drawn. Thereby, excessive flexion or bending of the alimentary canal 90 can be eliminated.

The above-mentioned procedure is repeated to simplify complicated bending or flexion of the alimentary canal 90 as shown in Fig. 5I. Accordingly, as shown in Fig. 5J, the insertion part 12 can be inserted into the deeper portion of the alimentary canal 90.

Next, a method of conveying a medical capsule 220 into the coelom using the endoscope device according to the embodiment of the invention will be described. In the following example, recovery of the medical capsule 220 from the deep alimentary canal, such as the small intestine, will be described.

While the hood 200 and the first balloon 60 are being attached to the forward end 44 of the insertion part 12, the insertion part 12 is inserted into the coelom. At this time, the operations shown in Figs. 5A to 5J may be performed as an insertion method, and the forward end 44 of the insertion part 12 is inserted into the deep alimentary canal, such as the small intestine.

After the forward end 44 of the insertion part 12 is inserted to a position of the medical capsule 220, the forward end 200A of the hood 200 approaches the medical capsule 220 in the coelom while an observation image, which is obtained using an observation optical system 52, is being observed.

Next, the suction button 30 is operated to start suctioning through the clamp opening 58. Thereby, gas (or liquid) is suctioned through the clamp opening 58 from the inside of the hood 200 to create a suction state in the hood 200. As shown in Fig. 4, since the inside of the hood 200 is in the suction state, the medical capsule 220 is suctioned to and held by the forward end 200A of the hood 200.

After the medical capsule 220 is held, the insertion part 12 of the endoscope 10 is drawn out from the coelom. Accordingly, the medical capsule 200 is conveyed from the coelom and recovered. During the conveying, since the medical capsule 220 is held in a range where the observation optical system 52 observes, the holding of the medical capsule 220 can be always observed using the observation image. Therefore, when the medical capsule 220 is fallen, the falling can be immediately checked.

When the insertion part 12 is drawn out from the coelom, the outside of the hood 200 can be observed because the hood 200 is transparent or semitransparent. Therefore, it is possible to avoid that the wall of the coelom catches the medical capsule 220. Thereby, the insertion part 12 is drawn out from the coelom to convey the medical capsule 220 to the outside of the coelom and to recover the capsule 220.

In the above-described embodiment, the medical capsule 220 is held and recovered out of the coelom, but the medical capsule 220 may be conveyed in the coelom and released. For example, in case the medical capsule 220 is caught in a narrow portion of coelom, r the medical capsule 220 is held and conveyed to the front of the narrow portion and then, the holding of medical capsule 220 may be released. The suction button 30 shown in Fig. 1 is operated to stop the suctioning through the clamp opening 58, to thereby release the holding of the medical capsule 220.

In addition to the holding of the medical capsule 220 in the coelom, the endoscope device may be used to hold the medical capsule 220 outside of the coelom, and convey the medical capsule 220 into the coelom by inserting the insertion part 12 into the coelom.

Next, the operation of the endoscope device according to the embodiment of the invention will be described.

As described above, the endoscope device of the embodiment of the invention is configured so that the base forward end 200A of the hood 200 can fix the forward end 60A of the first balloon 60. Therefore, unlike the case where an exclusive fixing unit fixes the forward end 60A of the first balloon 60 (see Fig. 10), the exclusive fixing unit and the hood 200 do not overlap in this embodiment. Accordingly, the diameter of the insertion part 12 after the hood is attached can be reduced. Hence, the insertion part 12 can be smoothly inserted into and drawn from the coelom and the insertion subsidiary member 70.

Moreover, according to the embodiment of the invention, since the forward end 60 A of the first balloon 60 is fixed by attaching the hood 200, the operation of attaching the hood 200 and the operation of fixing the first balloon 60 can be performed simultaneously to thus improve workability.

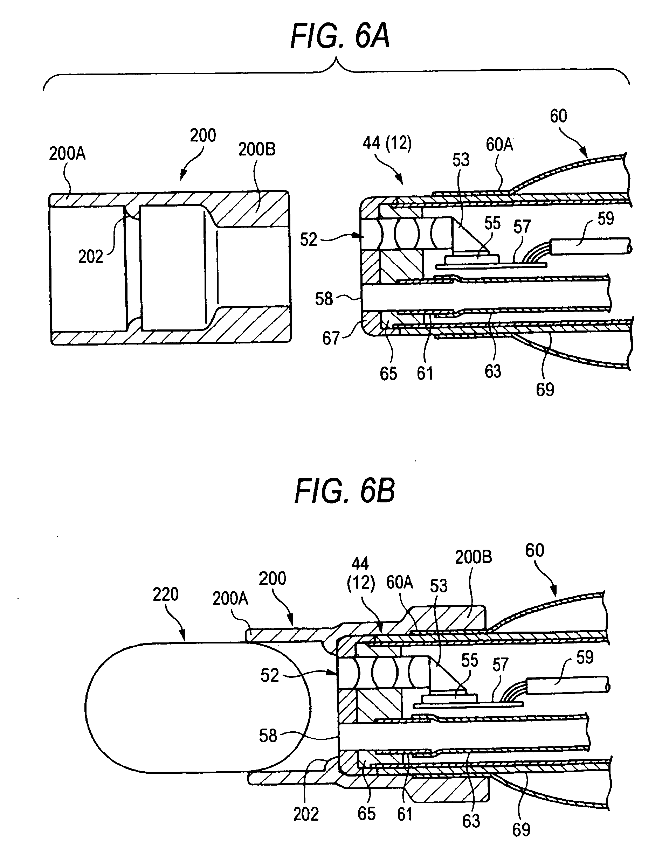

The shape of the hood 200 is not limited to the above-described embodiment. Particularly, the base end 200B of the hood member 200 may have any shape so long as the forward end 60 A of the first balloon 60 is fixed surely. For example, as shown in Fig. 6A, the base end 200B of the hood 200 may have a thick inner side so that its inner diameter is smaller than the other portions. In this case, as shown in Fig. 6B, while being enlarged, the narrow base end 200B is fitted to the forward end 44 of the insertion part 12, and fitted to the forward end 60A of the first balloon 60. This hood 200 can firmly fix the forward end 60A of the first balloon 60 because the base end 200B strongly tightens the forward end 60A of the first balloon 60.

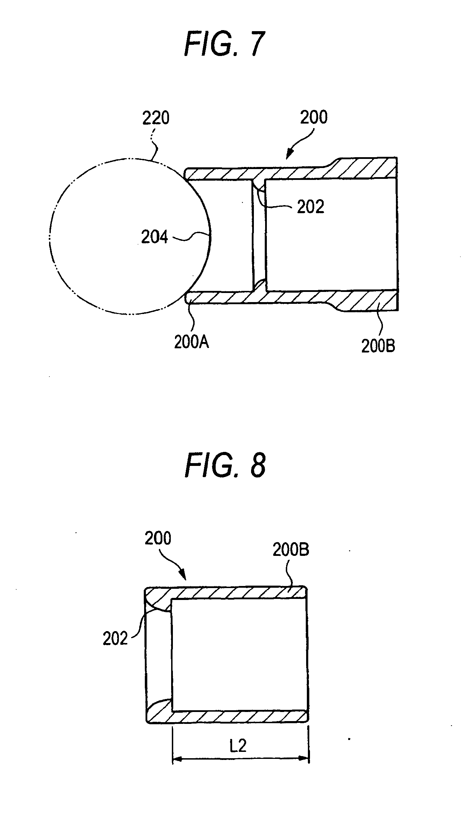

As shown in Fig. 7, the base end 200B of the hood 200 may have a thick outer side. In this case, since the force by which the hood 200 tightens the forward end 60A of the first balloon 60 is stronger, the hood 200 can fix the forward end 60A of the first balloon 60 firmly.

Additionally, the base end 200B of the hood 200 may be made of a material that is different from the material of the other portions. For example, the materials may be changed so that hardness of rubber of the base end 200B (that is, a portion which fixes the forward end 60A of the first balloon 60) is higher than that of the other portions.

The shape of the forward end 200A of the hood 200 is not limited to the above-mentioned embodiment. For example, as shown in Fig. 7, a groove 204 may be formed on the forward end 200A of the hood 200 to hold a lateral surface of the medical capsule 220 using the groove 204. Further, a tapered portion (not shown) may be formed on the inner surface of the forward end of the hood 200 shown in Fig. 3 so that a curved side of the medical capsule 220 is in surface-contact with the tapered portion and absorbed to and held by the tapered portion.

In the above-mentioned embodiment, a holder is provided on the forward end 200A of the hood 200 to hold the medical capsule 220, but the hood of the invention is not limited thereto. For example, as shown in Fig. 8, the hood 200 may not have the holder. Alternatively, the forward end of the hood 200 may be tapered.

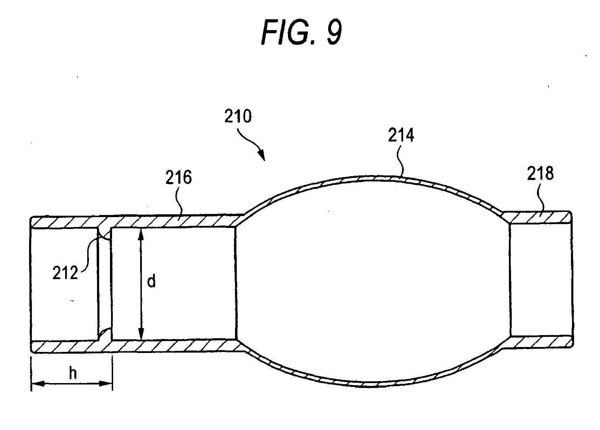

Next, a hood for an endoscope according to another embodiment of the invention will be described. A hood 210 shown in Fig. 9 has such a configuration that the hood 200 and the first balloon 60 shown Fig. 3 are formed integrally. That is, the hood 210 includes a balloon 214, which is thin, inflatable and deflatable; and thick fixing portions 216 and 218 formed on forward-end and base-end sides of the balloon 214. When the fixing portions 216 and 218 are fitted to an insertion part 12, the fixing portions 216 and 218 are fixed to a circumferential surface of the insertion part 12 firmly with being in close contact with the circumferential surface of the insertion part 12. Thus, even when air is supplied into the balloon 214 at a predetermined pressure, tight contact between the fixing portions 216 and 218 and the circumferential surface of the insertion part 12 is maintained.

A position regulation portion 212 protrudes from an inner surface of the hood 210 . A protrusion height h from the position regulation portion 212 to the forward-end side and an inner diameter d satisfy the above-mentioned equation (1). Accordingly, the medical capsule 220 can be held by the forward end of the hood 210.

With respect to the hood 210 having the above-mentioned configuration, since the balloon 214 is integrally formed with the hood, the hood 210 and the balloon 214 can be simultaneously attached.

According to the embodiment of the invention, since the fixing portions 216 and 218 fix both ends of the balloon 214, it is unnecessary to separately provide fixing members (for example, a band of Fig. 10) of the balloon 214, and the diameter of the insertion part 12 after the balloon can be reduced.

In the above-mentioned embodiment, the invention is applied to the double balloon type of endoscope device including the first balloon 60 and the second balloon 80. However, the endoscope device of the invention is not limited to the above-mentioned type as long as the first balloon 60 and the hood 200 are attached to the insertion part 12 of the endoscope 10. Accordingly, the invention may be applied to an endoscope device that does not have the second balloon 80, or an endoscope device that does not include the insertion subsidiary member 70.

## Claims

1. An endoscope (10) comprising a hood (200) fitted to an end (44) on an insertion part (12) of the endoscope and having a tube shape and further comprising a balloon (60) separate from the hood and attached to the insertion part (12) of the endoscope, **characterized in that** the hood comprises a balloon fixing part (200B) fitted to the outer surface of the forward end (60A) of the balloon (60), thereby tightening and fixing the balloon (60) to the insertion part (12), that the balloon fixing part (200B) is made of an elastic material, and that the balloon fixing part (200B) of the hood has an inner diameter, which is smaller than those of parts of the hood other than the balloon fixing part.

2. The endoscope according to claim 1, further comprising a holder at an end (200A) of the hood, wherein the holder can hold a medical capsule (220).

## Patentansprüche

1. Endoskop (10) mit einer auf ein Ende (44) eines Einführteils (12) des Endoskops aufgepassten Haube (200), die eine Rohrform aufweist, und weiterhin umfassend einen Ballon (60) getrennt von der Haube und an dem Einführteil (12) des Endoskops angebracht, **dadurch gekennzeichnet, dass** die Haube einen Ballon-Fixierteil (200B) aufweist, der auf die Außenfläche des vorderen Endes (60A) des Ballons (60) aufgesetzt ist, um dadurch den Ballon (60) an dem Einführteil (12) festzulegen und zu fixieren, dass der Ballon-Fixierteil (200B) aus einem elastischen Werkstoff besteht, und dass der Ballon-Fixierteil (200B) der Haube einen Innendurchmesser aufweist, der kleiner ist als jener der Teile der Haube ausgenommen den Ballon-Fixierteil.

**2.** Endoskop nach Anspruch 1, weiterhin umfassend einen Halter an einem Ende (200A) der Haube, wobei der Halter eine medizinische Kapsel (220) halten kann.

**Revendications**

**1.** Endoscope (10) comprenant un capuchon (200) monté sur une extrémité (44) sur une partie d'insertion (12) de l'endoscope et ayant une forme tubulaire et comprenant en outre un ballonnet (60) séparé du capuchon et fixé à la partie d'insertion (12) de l'endoscope, **caractérisé en ce que** le capuchon comprend une partie de fixation de ballonnet (200B) montée sur la surface externe de l'extrémité avant (60A) du ballonnet (60), serrant et fixant ainsi le ballonnet (60) sur la partie d'insertion (12), **en ce que** la partie de fixation de ballonnet (200B) est réalisée à partir d'un matériau élastique, et **en ce que** la partie de fixation de ballonnet (200B) du capuchon a un diamètre interne qui est plus petit que ceux des parties du capuchon différentes de la partie de fixation de ballonnet.

**2.** Endoscope selon la revendication 1, comprenant en outre un support au niveau d'une extrémité (200A) du capuchon, dans lequel le support peut supporter une capsule médicale (220).

FIG. 1

# FIG. 2

## FIG. 3

# FIG. 4

EP 1 759 627 B1

FIG. 5E

FIG. 5D

FIG. 5C

FIG. 5B

FIG. 5A

FIG. 5J

FIG. 5I

FIG. 5H

FIG. 5G

FIG. 5F

14

## FIG. 6A

## FIG. 6B

# FIG. 7

220    200

204    202

200A    200B

# FIG. 8

200    200B

202

L2

## FIG. 9

FIG. 10

**EP 1 759 627 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002301019 A
- US 5681260 A